**Europäisches Patentamt**

**European Patent Office**

**Office européen des brevets**

(19)

(11) Veröffentlichungsnummer : **0 211 229 B2**

(12) # NEUE EUROPÄISCHE PATENTSCHRIFT

(45) Veröffentlichungstag der neuen Patentschrift :
**14.12.94 Patentblatt 94/50**

(51) Int. Cl.⁵ : **G01N 33/53,** // G01N33/543,
C12Q1/68

(21) Anmeldenummer : **86108907.6**

(22) Anmeldetag : **01.07.86**

(54) Mit Überzügen versehene Formkörper zur Bindung von bioaffinen Substanzen.

(30) Priorität : **09.07.85 DE 3524451**

(43) Veröffentlichungstag der Anmeldung :
**25.02.87 Patentblatt 87/09**

(45) Bekanntmachung des Hinweises auf die
Patenterteilung :
**27.12.91 Patentblatt 91/52**

(45) Bekanntmachung des Hinweises auf die
Entscheidung über den Einspruch :
**14.12.94 Patentblatt 94/50**

(84) Benannte Vertragsstaaten :
**AT BE CH DE FR GB IT LI LU NL SE**

(56) Entgegenhaltungen :
EP-A- 0 071 704
EP-A- 0 163 799
EP-A- 0 174 247
DD-A- 236 400
DE-A- 2 847 995
DE-A- 3 321 629

(56) Entgegenhaltungen :
DE-A- 3 346 795
FR-A- 2 430 013
US-A- 4 357 142
US-A- 4 363 634
CHEMICAL ABSTRACTS, Band 98, Nr. 11, 14
März 1983, Columbus, OH (US); I.C.SHEKAR-
CHI et al., S. 243, Nr. 85532w
CLINICAL CHEMISTRY, Band 29, Nr. 5, Mai
1983, Washington, US; M.ISHAQ et al., Seiten
823-827
JOURNAL OF CLINICAL MICROBIOLOGY,
Band 16, Nr. 6, Dezember 1982; Seiten
1012-1018

(73) Patentinhaber : **BEHRINGWERKE
Aktiengesellschaft
Postfach 1140
D-35001 Marburg (DE)**

(72) Erfinder : **Franze, Reinhard, Dr.
Michaelsg. 2
D-3551 Lahntal-Sterzhausen (DE)**
Erfinder : **Beschle, Hans Georg, Dr.
Albert Demnitz-Weg 6
D-3550 Marburg (DE)**

EP 0 211 229 B2

## Beschreibung

Die Erfindung betrifft einen in den Ansprüchen im einzelnen näher gekennzeichneten Formkörper mit Überzügen aus Polymeren, wobei der in spezieller Weise hergestellte Überzug die Eigenschaft hat, biologische Substanzen mit bioaffinen Bindungseigenschaften zu binden, sowie ein Verfahren zu ihrer Herstellung. Diese mit Überzügen versehenen Formkörper können als "Träger" zur Bindung eines Partners eines bioaffinen Bindungspaares verwendet werden.

Für biologische Testverfahren werden oft Komponenten an einen festen Träger gebunden. um beispielsweise überschüssige Reaktionspartner leichter entfernen zu können. Trägergebundene Reaktionspartner werden beispielsweise in Radio- und Enzymimmunoassays verwendet. Zur Verbesserung der Haftung von biologischem Material an Kunststoffoberflächen sind solche Oberflächen bestrahlt worden. Weiterhin ist die Bindung von Testkomponenten an Träger durch Behandeln mit bifunktionellen chemischen Reagenzien wie Glutardialdehyd verbessert worden.

Es ist auch vorgeschlagen worden, zur Verringerung der Schwankungsbreite immunologischer Testverfahren Kunststoffträger zur Beschichtung mit immunologisch aktivem Material zu verwenden, die weniger als 1 Gewichtsprozent Hilfs- und Zusatzstoffe enthalten (EP-A 0 126 392).

Für Verfahren, an denen eine Nukleinsäurehybridierung beteiligt ist. sind zur Bindung einzelsträngiger Nukleinsäuren Membranen verwendet worden. Solche Membranen sind üblicherweise aus Nitrozellulose. Es ist auch bekannt, die Bindung von Nukleinsäuren an Kunststoffoberflächen durch Behandeln der Oberflächen mit Protamin zu fördern.

Jede dieser Methoden löst jeweils nur bestimmte Probleme in biologischen Bestimmungsverfahren, die mit trägergebundenen Komponenten arbeiten.

Es besteht deshalb weiterhin die Aufgabe, Verfahren zu finden, mit deren Hilfe die Haftung biologischen Materials an Trägeroberflächen verbessert werden kann.

Überraschenderweise wurde gefunden, daß durch Behandeln von Oberflächen von Formkörpern beispielsweise von Mikrotestplatten mit einer Lösung von Zellulosenitrat die Binaungseigenschaften dieser Formkörper für Nukleinsäuren oder Proteine oder sogar für Mikroorganismen wie Viren verbessert werden. Durch diese Behandlung war es überhaupt erst möglich geworden, Nukleinsäuren an Polystyrol zu binden, wie es für die Herstellung von Mikrotestpiatten häufig verwendet wird.

Gegenstand der Erfindung sind Platten mit konkaven Vertiefungen und Röhrchen, die gleichzeitig zur Aufnahme von Lösungen dienen, aus einem Polymer mit einem Überzug aus einem zweiten Polymer, wobei die Kombination für wässrige Lösungen undurchlässig ist und der Überzug aus einem Polymer besteht oder ein solches enthält, das die Eigenschaft besitzt, aus wässrigen Lösungen eine biologische Substanz, die ein bioaffiner Bindungspartner ist, zu binden und dieses zweite Polymer eine derivatisierte Zellulose ist, wobei der Überzug dadurch erhalten wird, daß das Beschichtungsmaterial in einem Lösungsmittel, das das Formkörpermaterial nicht beeinträchtigt, gelöst wird, in die jeweiligen Vertiefungen bzw. Röhrchen eingebracht und nach einer Zeit abgedampft wird.

Die Formkörper bestehen aus Kunststoffen. Sie sind plane Plättchen, Platten mit konkaven Vertiefungen zur Aufnahme von Lösungen, wie Mikrotestplatten oder Röhrchen.

Der Überzug besteht aus einem Polymeren, der einmal die Eigenschaften hat, an dem Material des Formkörpers zu haften, zum anderen biologische Materialien aus wässrigen Lösungen zu binden in einer Weise, daß sie als Reaktionspartner von bioaffinen Bindungssystemen verfügbar bleiben. Der Verbund aus einem Formkörper und einem Überzug ist für wässrige Lösungen undurchlässig.

Der Überzug kann aus dem genannten Polymer oder aus einer Mischung die diesen Polymer enthält bestehen. Er kann Zusätze enthalten, welche einerseits die Anhaftung des Überzugs an den Formkörper und andererseits die Bindung der bioaffinen Komponenten begünstigen.

Wie oben erwähnt, werden als Polymere derivatisierte Zellulosen, wie Zellulosenitrate. davon insbesondere Kollodiumwollen, sowie Acetylzellulose verwendet. Es werden Polymere bevorzugt, die die optischen Eigenschaften des Formkörpers, beispielsweise die Transparenz oder die Reflektion nicht oder nur wenig verändern.

Die polymerhaltige Lösung oder Dispersion werden in die Röhrchen oder die Vertiefungen eingefüllt und anschließend darin belassen und danach wird das Lösungsmittel bzw, das Dispergiermedium abgedampft. Die Art der Trocknung richtet sich nach den Verdampfungseigenschaften des jeweils verwendeten Lösungsmittels oder Dispergiermediums.

Vorzugsweise besteht der Überzug aus oder enthält Zellulosenitrat.

Als Lösungsmittel ist im Falle von Zellulosenitrat beispielsweise Ethanol geeignet, doch sind auch andere Lösungsmittel verwendbar. Wesentlich ist, daß der Formkörper durch das Lösungsmittel nicht in einer Weise verändert wird, daß seine Brauchbarkeit in Tests beeinträchtigt ist.

Wird eine Lösung von Zellulosenitrat verwendet, beispielsweise in Ethanol, so liegt die Konzentration vorzugsweise zwischen 1 µg/ml und 20 mg/ml, vorzugsweise 2 µg/ml bis 2 mg/ml Zellulosenitrat.

Die biologischen Materialien mit bioaffinen Bindungseigenschaften können sein Proteine oder Glycoproteine, die die Funktion von Antigenen, von Antikörpern, hier sowohl polyklonale wie monoklonale, und von Lektinen haben. Weiterhin könne Lipoproteine, Glycolipide, Oligo- und Polysaccharide mit immmunologischen oder mit nicht immunologischen Bindungseigenschaften an die Polymerschicht gebunden werden. Einzelsträngige Desoxyribonukleinsäure (DNS) oder einzelsträngige Ribonukleinsäure (RNS), sowie deren Oligonukleotide werden ebenfalls an den Überzug gebunden, wodurch die derart beschichteten Flächen für Hybridisierungstechniken verwendbar werden.

Für die zur Bindung an den Überzug vorgesehenen biologischen Materialien sind wässrige Lösungen sowie Pufferlösungen geeignet, in der diese gelöst oder dispergiert sind; dispergiert, wenn es sich um Glycolipide, Lipoproteine, Viren, Zellen sowie Bestandteile von Zellen eucaryoter oder procaryoter Struktur handelt. Die Lösungen können Zusätze enthalten, die die gleichmäßige Bindung der biologischen Materialien gewährleisten.

Die Konzentration des an den Überzug zu bindenden, jeweiligen bioloigschen Materials und die Lösung, beziehungsweise deren Pufferkomponenten, deren Ionenstärke und deren pH-Wert, und die Bedingungen, wie Temperatur und Zeitdauer des Zusammenbringens der Flüssikgkeit, die das zu bindende biologische Material enthält, und des mit dem Überzug versehenen Formkörpers werden im allgemeinen zum Erreichen einer gleichmäßigen und hohen Besetzung mit möglichst irreversibler Bindung des jeweiligen biologischen Materials an die Oberfläche in Vorversuchen ermittelt. Die Konzentrationen der biologischen Materialien liegen beispielsweise zwischen 5 mg/ml und 5 ng/ml oder auch niedriger. Als Puffer werden beispielsweise Phosphat-, Tris/HCl- und Carbonat-Puffer in Konzentrationen von 10 bis 100 mM und der pH-Bereich von 7 bis 10, vorzugsweise 8,0 bis 9,6 verwandt. Die Zeit kann variiert werden zwischen 30 min und 20 h, die Temperatur zwischen 4° C und 80° C. Eine der möglichen Arbeitsweisen für solche Vorversuche ist für die Bindung von Proteinen an Polystyrol von Cantarero et al. (1980), Anal. Biochem. 105, 375-382, beschrieben.

Die mit Überzügen versehenen Formkörper können zur Herstellung eines analytischen Mittels verwendet werden.

Die Erfindung wird durch folgende Beispiele näher erläutert, ohne damit den Erfindungsgegenstand einzugrenzen.

Beispiel

1. Beschichtung von Mikrotestplatten mit Zellulosenitrat als Polymer

Mikrotestplatten aus Polystyrol (A/S Nunc, 4000 Roskilde, Dänemark, Kat.Nr. 262170, mit 96 Vertiefungen und rundem Boden, Qualität Standard hygienisch) wurden verwandt.

Es wurden Lösungen von Zellulosenitrat (NCA 400 und NCA 500 der Fa. Wolff Walsrode AG, 3030 Walsrode, Bundesrepublik Deutschland) in absolutem Ethanol in folgenden Konzentrationen hergestellt: 2 mg/ml und 50 µg/ml NCA 400, sowie 100 µg/ml NCA 500. Alle Vertiefungen von mehreren Mikrotestplatten wurden mit jeweils 100 µl einer Lösung der genannten Konzentrationen und als Kontrolle nur mit dem Lösungsmittel Ethanol gefüllt. Das Lösungsmittel wurde anschließend durch Überblasen eines Luftstroms abgedampft.

2. Verwendung von mit einem Überzug aus Zellulosenitrat beschichteten Mikrotestplatten für einen Enzymimmmunoassay (ELISA) zum Nachweis von Antikörpern gegen Mumps.

2.1. Bindung von Mumpsantigenen an die beschichteten und unbeschichteten Mikrotestplatten

Mumpsvirus, gezüchtet in Verozellen, und Verozellen, die nicht mit Mumpsvirus infiziert waren, wurden nach Nicolai-Scholten et al. (1980), Med.Microbiol. Immunol. 168, 81-90, zu Präparationen aufgearbeitet, die im weiteren als Mumpsantigen und als (negatives) Kontrollantigen bezeichnet werden.

In alle 192 Vertiefungen von jeweils 2 mit 100 µg/ml Zellulosenitrat NCA 500 in Ethanol beschichteten und von jeweils 2 nur mit Ethanol behandelten Mikrotestplatten (siehe Beispiel 1) wurden gemäß Popow-Kraupp et al. (1982), J.Med.Virol. 10, 119-129 und gemäß Sakata et al. (1984), J.Clin.Microbiol. 19, 21-25, jeweils 2 mal 48 50 µl Aliquote der Lösungen von Mumpsantigen und von Kontrollantigen eingefüllt derart, daß die mit den beiden Lösungen beschickten Vertiefungen alternierend nebeneinanderlagen. Es wurde 15 bis 20 h bei 4° C inkubiert. Anschließend wurden die Lösungen abgesaugt und die Vertiefungen gewaschen.

2.2. Nachweis von Mumpsantikörpern

Die Testdurchführung erfolgte im wesentlichen wie von Nicolai-Scholten et al. beschrieben. Ein positives Kontrollserum wurde 1:1000 verdünnt mit einer Lösung von 1 % Rinderserum, 4 % Polyoxiethylen-(20)-sorbitanmonolaureat (Tween ® 20) in 130 mM NaCl, 20 mM Natriumphosphat, pH 7,2 (PBS). Davon wurden 100 µl in alle 96 Vertiefungen pro Mikotestplatte gegeben. Es wurde 1 h bei 37° C und bei gesättigter Luftfeuchtigkeit inkubiert, anschließend dreimal mit jeweils 300 µl einer Lösung von 1 % Tween ® 20 in PBS gewaschen. Es folgte eine Inkubation, d.h. 1 h bei 37° C, mit 50 µl pro Vertiefung der von Nicolai-Scholten beschribenen Lösung des Konjugates von Anti-human IgG mit Alkalischer Phosphatase (AP). Anschließend wurde wieder dreimal mit jeweils 300 µl der Lösung von 1 % Tween ® 20 in PBS gewaschen. Die enzymatische Aktivität des in den Vertiefungen gebundenen AP-Konjugates wurde bestimmt durch Inkubation mit 100 µl pro Vertiefung einer Lösung von 1,5 g/l p-Nitrophenylphosphat in 10 % Diethanolamin in Wasser, eingestellt mit HCl auf pH 9,8 bei 20 bis 25° C während 45 min. Die Reaktion wurde durch Zugabe von 50 µl pro Vertiefung einer Lösung von 2 N NaOH beendet. Die Extinktionen der gefärbten Lösungen wurden bei 405 nm mit dem Gerät Titertek ®, Multiskan MC, einem Photometer der Firma Flow Laboratories Inc., McLean, Va. 22102, USA, gemessen.

2.3. Ergebnis

In Tabelle 1 ist das Ergebnis dargestellt. Die Extinktionen der mit Mumpsantigen beladenen Vertiefungen bei den mit Zellulosenitrat behandelten Platten sind etwa um das dreifache höher als bei den nur mit dem Lösungsmittel Ethanol behandelten Platten. Die mit dem (negativen) Kontrollantigen behandelten Vertiefungen zeigen sowohl im Falle der Verwendung von der mit Zellulosenitrat behandelten als auch von nur mit dem Lösungsmittel behandelten Platten eine niedrige Extinktion, die im ersten Fall zwischen 0,01 und 0,02 und im zweiten zwischen 0 und 0,01 liegt. Bei den mit Zellulosenitrat behandelten Platten ist diese als Hintergrund zu bezeichnende Extinktion als sehr niedrig zu bewerten, ein Zeichen für geringe unspezifische Bindung des Kontrollserums wie des AP-IgG-Konjugates. Hervorzuheben ist die drei- mal höhere Extinktion bei den beschichteten Platten, was eine Erhöhung der Empfindlichkeit um die gleiche Größenordnung bedeutet.

**Tabelle 1**

| Mikrotestplatte Überzug von Zellulosenitrat in µg pro Vertiefung | Mittlere Extinktions- und Standardabweichung bei 405 nm von Vertiefungen behandelt mit | | | | Extinktionsdifferenz von Vertiefungen Mumpsantigen-Kontrollantigen | |
|---|---|---|---|---|---|---|
| | Mumpsantigen | | Kontrollantigen | | | |
| | Platte 1 n = 48 | Platte 2 n = 48 | Platte 1 n = 48 | Platte 2 n = 48 | Platte 1 | Platte 2 |
| 10 | 0,410 ± 0,041 | 0,350 ± 0,030 | 0,020 | 0,010 | 0,390 ± 0,040 | 0,340 ± 0,031 |
| 0 | 0,150 ± 0,017 | 0,120 ± 0,015 | 0,010 | 0 | 0,140 ± 0,017 | 0,120 ± 0,015 |

3. Verwendung von mit Überzügen aus Zellulosenitrat beschichteten Mikrotestplatten für die DNS-Hybridisierung

3.1. Bindung von einzelsträngiger DNS an die beschichtete Mikrotestplatte

Es wurden Mikrotestplatten verwandt, deren Vertiefungen wie in Beispiel 1 beschrieben, mit Lösungen von jeweils 2 mg/ml und 50 µg/ml Zellulosenitrat NCA 400 und zur Kontrolle nur mit dem Lösungsmittel Ethanol behandelt worden waren.

In die Vertiefungen der beschichteten und in die der als Kontrollen nur mit Ethanol behandelten Vertiefungen wurden 100 µl von Verdünnungen von 500, 250, 50, 5 und 0 ng/ml DNS des Plasmids, pBW3 in Puffer von 10 mM Tris/HCl, 1 mM EDTA, pH 8,0, die durch 10 min Erhitzen auf 100° C, gefolgt von 5 min Kühlen auf 0° C, in Einzelstränge zerlegt worden war, eingefüllt. Die mit den Lösungen beladenen Platten wurden bei 80° C 8 h lang gehalten (gebacken), wobei sie zur Trockene eindampften.

3.2. Vorhybridisierung

Als Stammlösungen für die Herstellung von im folgenden aufgeführten Puffern wurden 50fach konzentrierte Denhardt'sche Lösung (Denhardt, 1966, Biochem.Biophys. Res.Comm. 23, 641-652): 10 g Ficoll ® 400 (ein Saccharose-Polymer der Fa. Pharmacia, Mg 400.000), 10 g Polyvinylpyrrolidon, MG 360.000, 10 g Rinderserumalbumin, gelöst in 1 l destilliertem Wasser und 20fach konzentrierte Citratgepufferte Kochsalzlösung (SSC, Abkürzung für Standard sodium citrate), das heißt 3,0 M NaCl, 300 mM Trinatriumcitrat verwandt.

Für die Vorhybridisierung zur Abdeckung unspezifischer Bindungsstellen wurden alle Vertiefungen mit jeweils 100 µl folgender Lösung gefüllt und 4 h bei 42°C inkubiert: 0,01 % Hefe RNS, 0,1 % Natriumdodecylsulfat (SDS), 1 % Glycin, 5-fach konzentrierte Denhardt'sche Lösung, 5-fach konzentrierte SSC, 50 % Formamid in 50 mM Natriumphosphatpuffer, pH 7,0.

3.3. Hybridisierung

Als Sonden-DNS wurde durch "Nick"-Translation biotinierte DNS des Plasmid pBW3 verwandt, hergestellt wie von Rigby et al. (1977), J.Mol.Biol. 113, 237-251, beschrieben, die auf die Konzentration von 250 ng/ml verdünnt worden war mit einer Lösung von 0,01 % Hefe-RNS, 0,1 % SDS, 10 % Dextransulfat, einfach konzentrierter Denhardt'scher Lösung, 5-fach konzentrierter SSC, 45 % Formamid in 20 mM Natriumphosphat-Puffer, pH 7,0. Die gebrauchsfertige Sondenlösung wurde erhalten, indem sie 10 min bei 100° C erhitzt und anschließend auf 0° C abgekühlt wurde.

In jede Vertiefung wurden 100 µl DNS-Sondenlösung eingefüllt. Zur Hybridiserung wurde 15 h bei 42° C inkubiert.

3.4. Detektion

Die Sondenlösung wurde abgesaugt. Es folgten mehrere Waschschritte mit in der Reihenfolge angegebenen Mengen der folgenden Lösungen, den angegebenen Inkubationszeiten und Inkubationstemperaturen:

3mal jeweils 3 min bei 20 bis 25° C mit 300 µl 0,1 % SDS in 2-fach konzentrierter SSC,

3mal jeweils 3 min bei 20 bis 25° C mit 300 µl 0,1 % SDS in 0,2-fach konzentrierter SSC,

2mal jeweils 15 min bei 50° C mit 300 µl 0,1 % SDS in 0,16-facher SSC und

3mal jeweils 1 min bei 20-bis 25° C mit 150 µl 0,1 % SDS in 2-facher SSC.

Anschließend wurde jeweils einmal mit jeweils 300 µl pro Vertiefung der im folgenden als Puffer A und als Puffer B bezeichneten Lösungen behandelt, um unspezifische Bindung von Protein an die Festphase zu vermeiden.

Puffer A: 0,05 % 4-Octylphenoxipolyethoxiethanol (Triton® X 100), 0,1 M Tris/HCl, 0,1 M NaCl, 2 mM MgCl$_2$, pH 7,5, 1 min bei 20 bis 25° C;

Puffer B: 3 % Rinderserumalbumin in Puffer A, 20 min bei 42° C.

Es folgte eine Inkubation, das heißt eine Wärmebehandlung bei 80° C für 10 min ohne Puffer.

Zur Detektion wurden die Vertiefungen erst wieder mit 300 µl Puffer B gefüllt, die Flüssigkeit 20 min bei 20 bis 25° C darin belassen und danach wieder abgesaugt.

100 µl pro Vertiefung einer Lösung von 2 µg/ml Streptavidin (Bethesda Research Laboratories, Gaithersburg, Ma. 20877, USA) in Puffer A wurden 1 h bei 20 bis 25° C inkubiert. Danach wurde dreimal zu jeweils 2 min mit 300 µl Puffer A gewaschen. Es folgte die Inkubation von 100 µl pro Vertiefung DNA Detection System in einer Konzentration von 1 µg/ml in Puffer A 1 h bei 20 bis 25° C. DNA-Detection System (Bethesda Res.Labs.) ist ein Polymer enthaltend biotinierte alkalische Phosphatase, hergestellt wie von Leary et al. (1983), Proc.Nat.Acad.Sci. 80, 4045-4049, beschrieben. Anschließend wurde zur Entfernung von nicht gebundenem DNA-Detection System jeweils dreimal 2 min bei 20-25° C mit 300 µl folgender zwei Pufferlösungen gewaschen:

Puffer A

und 0,1 M Tris/HCl, 0,1 M NaCl, 50 mM MgCl$_2$, pH 9,5.

Mit 100 µl pro Vertiefung einer Lösung von 1,5 mg/ml p-Nitrophenylphosphat in 10 % Diethanolamin in Wasser, eingestellt mit HCl auf pH 9,8, als Substrat für die alkalische Phosphatase wurde bei 37° C 45 min lang inkubiert und danach die enzymatische Hydrolyse durch Zugabe von 50 µl pro Vertiefung 2 N Natronlauge beendet. Die Extinktionen der gefärbten Lösungen wurden bei 405 nm in den Vertiefungen ebenfalls mit dem Gerät Titertek ® Multiskan MC gemessen.

3.5. Ergebnis und Bewertung des Ergebnisses

In Tabelle 2 ist das Ergebnis dargestellt. Vertiefungen, die wie bei 3.1. beschrieben mit 2mg/ml und

mit 50 μg/ml Zellulosenitrat NCA 400 beschichtet und anschließend mit einzelsträngiger DNS des Plasmids pBW3 der Konzentrationen 5 bis 500 ng/ml durch Backen behandelt worden waren, erzeugen nach der Hybridisierung und Bindung des DNS-Detektionssystem an die Sonde Farbsignale, die mit steigender DNS-Konzentration zunehmen. Das Farbsignal des Hintergrunds, das heißt das in den Vertiefungen, die nicht mit DNS behandelt worden waren, ist niedriger und auch etwa so schwach, wie das in den Vertiefungen, die nicht mit Zellulosenitrat beschichtet, aber mit DNS der genannten Konzentrationen behandelt worden waren (Kontrollen). Das Ergebnis zeigt, daß ohne Beschichtung mit dem Überzug keine DNS nachweisbar ist.

**Tabelle 2**

Extinktionen bei 405 nm nach DNS-Hybridisierung und Detektion mit AP Detektionssystem für DNS bei vorangegangener Behandlung (Backen) mit einzelsträngiger DNS des Plasmids pBW3 mit den Mengen

| Mikrotestplatte: Überzug von zellulosenitrat in µg/Vertiefung | | |
|---|---|---|
| 200 | 5 | 0 |
| 0,10 | 0,10 | 0,15 |
| 0,25 | 0,25 | 0,15 |
| 0,62 | 0,69 | 0,19 |
| 1,61 | 1,40 | 0,15 |
| 2,31 | 1,92 | 0,15 |

ng pro Vertiefung: 0, 0,5, 5, 25, 50

## Patentansprüche

1. Platten mit konkaven Vertiefungen und Röhrchen, die gleichzeitig zur Aufnahme von Lösungen dienen, aus einem Polymer mit einem Überzug aus einem zweiten Polymer, wobei die Kombination für wässrige

8

Lösungen undurchlässig ist und der Überzug aus einem Polymer besteht oder ein solches enthält, das die Eigenschaft besitzt, aus wässrigen Lösungen eine biologische Substanz, die ein bioaffiner Bindungspartner ist, zu binden und dieses zweite Polymer eine derivatisierte Zellulose ist, dadurch gekennzeichnet, daß der Überzug dadurch erhalten worden ist, daß das Beschichtungsmaterial in einem Lösungsmittel, das das Formkörpermaterial nicht beeinträchtigt, gelöst, in die jeweiligen Vertiefungen bzw. Röhrchen eingebracht und nach einer Zeit abgedampft wurde.

## Claims

1. Plates with concave depressions and tubes, which are simultaneously used to receive solutions, composed of a polymer with a coating of a second polymer, the combination being impermeable to aqueous solutions and the coating being composed of or containing a polymer which has the property of binding, from aqueous solutions, a biological substance which is a partner in bioaffinity binding, and this second polymer being a derivatized cellulose, wherein the coating has been obtained by the coating material having been dissolved in a solvent which does not adversely affect the material of the shaped article, introduced into the particular depressions or tubes and, after a period of time, evaporated.

## Revendications

1. Plaques comprenant des cavités et des petits tubes, servant simultanément à l'absorption de solutions, constituées d'un polymère revêtu d'un second polymère, laquelle combinaison est imperméable aux solutions aqueuses dans lesquelles le revêtement consiste en un polymère ou en contient un, qui présente la propriété de lier une substance biologique à partir de solutions aqueuses, laquelle constitue un partenaire de liaison bioaffin, ce deuxième polymère étant un dérivé de la cellulose, caractérisées en ce que le revêtement est obtenu en dissolvant le matériau de revêtement dans un solvant qui n'altère pas le matériau de la pièce façonnée, en l'introduisant dans chaque cavité ou tube et en le séchant par évaporation après un certain temps.